# EUROPEAN PATENT SPECIFICATION

(11) **EP 2 604 240 B1**
(45) Date of publication and mention of the grant of the patent: **06.08.2014**
(21) Application number: 12197364.8
(22) Date of filing: 14.12.2012
(51) Int. Cl.: A61F 13/535

(54) **Absorbent article including an absorbent core layer having a material free zone and a transfer layer arranged below the absorbent core layer**
Aufnahmefähiger Artikel mit aufnahmefähiger Kernschicht mit materialfreier Zone und Transferschicht unterhalb der aufnahmefähigen Kernschicht
Article absorbant comprenant une couche centrale absorbante ayant une zone dépourvue de matériau et une couche de transfert disposée en dessous de la couche centrale absorbante

(30) Priority: 15.12.2011 US 201113326725
(43) Date of publication of application: 19.06.2013
(73) Proprietor: McNeil-PPC, Inc., Skillman, New Jersey 08558 (US)
(72) Inventor: Bissah, Kofi, Somerset, NJ New Jersey 08873 (US); Hernandez, Francisco J. V., 12242-530 São Paulo (BR); Paques, Fernanda Wiermann, 12242-431 São Paulo (BR); Poccia, John, Monmouth Beach, NJ New Jersey 07750 (US)
(74) Representative: Carpmaels & Ransford LLP

(56) References cited:
- WO-A1-01/97736
- WO-A1-95/17869
- US-A- 4 973 325
- US-A- 5 662 633
- US-A- 5 954 705

## Description

### FIELD OF INVENTION

The present invention generally relates to absorbent sanitary napkins and in particular to a sanitary napkin that has superior transverse and longitudinal wicking characteristics, as well as superior fluid penetration time and rewet properties.

### BACKGROUND OF THE INVENTION

In order for a sanitary napkin to efficiently absorb a large amount of fluid during use it must effectively wick fluid throughout the absorbent structure of the napkin. Absent effective wicking properties menstrual fluid tends to pool in certain regions of the napkin as a result of which the full absorbent capacity of the napkin is not effectively utilized. Accordingly, the inventors of the present invention have recognized a need to provide a sanitary napkin that efficiently wicks fluid in the longitudinal and transverse directions of the napkin to thereby fully utilize the full absorbent capacity of the napkin, while also providing superior fluid penetration time and rewet properties.

### SUMMARY OF THE INVENTION

In view of the foregoing, the present invention provides an absorbent article including a longitudinally extending centerline, a transversely extending centerline, a liquid permeable cover layer having a body facing surface, a liquid impermeable barrier layer, an absorbent core arranged adjacent to the cover layer, the absorbent core including an upper surface and a lower surface and at least one material-free zone extending from the upper surface to the lower surface, a transfer layer arranged between the core and the barrier layer, the transfer layer including a planar portion having an upper surface and a lower surface and at least one protrusion extending upwardly from the upper surface, wherein the cover layer includes at least one first region arranged in spaced relationship to the transfer layer and at least one second region arranged in surface to surface contact with the transfer layer, wherein the at least one protrusion is structured and arranged to be received within, and extend upwardly into the at least one material free zone, and wherein the at least one protrusion has a height that is greater than a distance between the upper surface of the absorbent core and the lower surface of the absorbent core.

The present invention further provides an absorbent article including a longitudinally extending centerline, a transversely extending centerline, a liquid permeable cover layer having a body facing surface, a liquid impermeable barrier layer, an absorbent core arranged adjacent to the cover layer, the absorbent core including an upper surface and a lower surface and a material-free zone extending from the upper surface to the lower surface, a transfer layer arranged between the core and the barrier layer, the transfer layer including a planar portion having an upper surface and a lower surface and a protrusion extending upwardly from the upper surface, wherein the cover layer includes a first region arranged in spaced relationship to the transfer layer and a second region arranged in surface to surface contact with the transfer layer, wherein the protrusion is structured and arranged to be received within, and extend upwardly into the material free zone, and wherein the protrusion has a height that is greater than a distance between the upper surface of the absorbent core and the lower surface of the absorbent core.

The material-free zone may be centrally aligned with respect to the longitudinally extending centerline and the transversely extending centerline. The material-free zone may extend over an area between about 400 mm² and about 6000 mm². The material-free zone may be substantially elliptical in shape and may have a length as measured along the longitudinally extending centerline in the range of about 40 mm to about 250 mm, or about 40mm to about 160mm, and a width as measured along the transversely extending centerline of about 10 mm to about 60 mm.

The protrusion may extend over an area between about 400 mm² and about 6000 mm². The protrusion may have a height in the range of between about 0.5 mm and about 50 mm, or in the range of between about 1.0 mm and about 35 mm, or in the range of between about 1.5 mm and about 30 mm. The protrusion may be generally elliptical in shape.

The present invention further provides an absorbent article including a longitudinally extending centerline, a transversely extending centerline, a liquid permeable cover layer having a body facing surface, a liquid impermeable barrier layer, an absorbent core arranged adjacent to the cover layer, a transfer layer including a planar portion having an upper surface and a lower surface and a plurality of protrusions extending upwardly from the upper surface, wherein the absorbent core includes an upper surface and a lower surface, the absorbent core comprising a plurality of beams and a plurality of material-free zones, each of the beams arranged in a spaced relationship to an adjacent beam and each of the beams being separated from an adjacent beam by a material-free zone, each of the material-free zones extending from the upper surface to the lower surface, wherein the cover layer includes a plurality of first regions arranged in spaced relationship to the transfer layer and a plurality of second regions, each of the second regions located between two adjacent beams and arranged in surface to surface contact with the transfer layer, wherein each one of the plurality of protrusions is structured and arranged to be received within, and extend upwardly into one of the plurality of material free zones, and wherein each protrusion has a height that is greater than a distance between the upper surface of the absorbent core and the lower surface of the absorbent core. The transfer layer may be arranged between the core and the barrier layer.

Each of the material-free zones may have a width in the range of between about 1 mm and about 10 mm and a length in the range of between about 40 mm and about 250 mm, or between about 50 mm and about 250 mm. Each of the material-free zones may extend over a surface area in the range of between 50 mm² and about 4000 mm². The material-free zones may be linear in shape, parallel to each other, and equally spaced.

The absorbent article may include between 2 and about 7, or between 2 and 7, longitudinally extending material-free zones and each of the material free zones may be spaced from an adjacent material-free zone in the transverse direction by a distance of from about 5 mm to about 30 mm.

Each protrusion may have a width in the range of between about 1 mm and about 10 mm and a length in the range of between about 40 mm and about 250 mm, or between about 50 mm and about 250 mm. The protrusions may be linear in shape, parallel to each other, and equally spaced. The absorbent article may include between 2 and about 7, or between 2 and 7, protrusions and each protrusion may be spaced from an adjacent protrusion in the transverse direction by a distance from about 5 mm to about 30 mm.

Each of the protrusions may extend over a surface area in the range of between 50 mm² and about 4000 mm². Each protrusion may have a height in the range of between about 0.5 mm and about 50 mm, or in the range of between about 1.0 mm and about 35 mm, or in the range of between about 1.5 mm and about 30 mm.

### BRIEF DESCRIPTION OF THE DRAWINGS

Examples of embodiments of the present invention will now be described with reference to the drawings, in which:
Fig. 1 is a top perspective view of an absorbent article according to the present invention;
Fig. 2 is a bottom perspective view of the absorbent article shown in Fig. 1;
Fig. 3 is an exploded view of the absorbent article shown in Fig. 1 according to a first embodiment of the invention;
Fig. 4 is sectional view of the absorbent article shown in Fig. 1 taken along line 4-4 in Fig. 1;
Fig. 5 is partially cut-away perspective view of the absorbent article shown in Fig. 1 schematically depicting the path of fluid flow within the article;
Fig. 6 is an exploded view of the absorbent article shown in Fig. 1 according to a second embodiment of the invention;
Fig. 7 is a sectional view of the absorbent article shown in Fig. 6 taken along line 7-7 in Fig. 6;
Fig. 8 is a top perspective view of an absorbent article according to a third embodiment of the present invention;
Fig. 9 is an exploded view of the absorbent article shown in Fig. 8;
Fig. 10 is a sectional view taken of the absorbent article shown in Fig. 8 taken along line 10-10 in Fig. 8;
Fig. 11 is a top perspective view of an absorbent article according to a fourth embodiment of the present invention;
Fig. 12 is an exploded view of the absorbent article shown in Fig. 11;
Fig. 13 is a sectional view of the absorbent article shown in Fig. 11 taken along line 13-13 in Fig. 11;
Fig. 14 is a top perspective view of an absorbent article according to a fifth embodiment of the present invention;
Fig. 15 is an exploded view of the absorbent article shown in Fig. 14;
Fig. 16 is a sectional view of the absorbent article shown in Fig. 15 taken along line 16-16 in Fig. 14.

### DETAILED DESCRIPTION OF THE INVENTION

The present invention generally relates to disposable absorbent articles such as sanitary napkins, pantiliners, absorbent products for incontinence, and other disposable absorbent articles worn close to a wearer's body. Although the invention will be described herein with reference to a sanitary napkin, the invention may be utilized with other disposable sanitary absorbent articles such as absorbent products for incontinence, diapers, pantiliners and the like.

Absorbent articles according to the present invention provide superior fluid handling characteristics, and more specifically provide superior longitudinal and transverse wicking characteristics, as well as superior fluid penetration time and rewet properties.

In the present disclosure, where reference is made to a range of values of "between x and y" the range of values may be inclusive of x and y. Alternatively, the range of values may be exclusive of x and y.

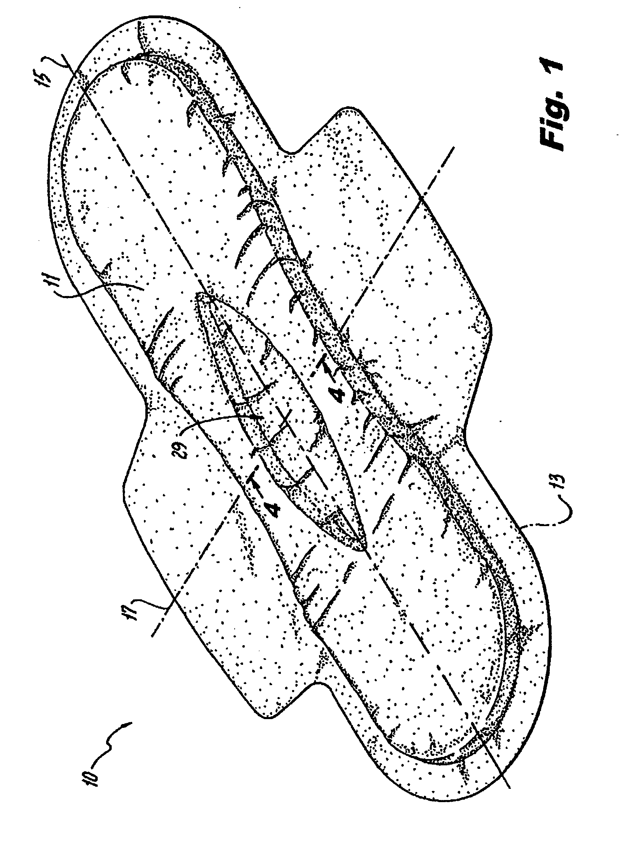

As shown in Figs. 1-4, the present invention relates to a sanitary napkin 10 for absorbing bodily fluids. The sanitary napkin 10 includes a body facing surface 11, a garment facing surface 13, a longitudinally extending centerline 15, and a transversely extending centerline 17.

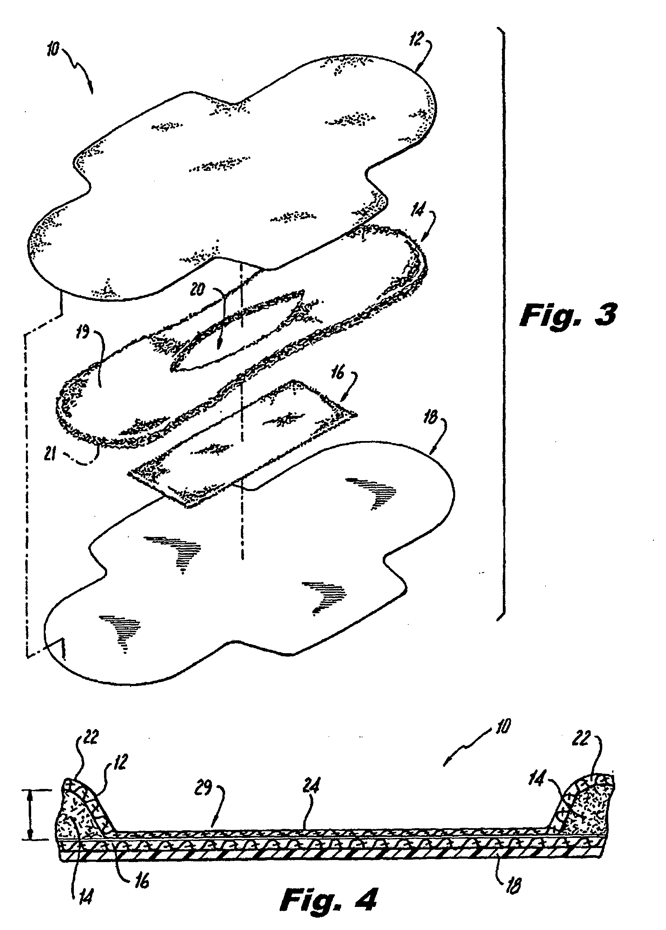

As best seen in the exploded view shown in Fig. 3, the sanitary napkin 10 includes a fluid permeable cover layer 12, an absorbent core 14, a transfer layer 16, and a fluid impermeable barrier layer 18. As shown in Fig. 3, the absorbent core 14 is arranged adjacent to the cover layer 12 and the transfer layer 16 is arranged between the absorbent core 14 and the barrier layer 18.

The absorbent core 14 includes a material-free zone 20 that is devoid of any absorbent material. The material-free zone 20 extends from an upper surface 19 of the absorbent core 14 to a lower surface 21 of the absorbent core 14. The material-free zone 20 may be formed by any known method such as cutting or the like. In the specific embodiment of the invention shown in Figs. 1-4 the material-free zone 20 is centrally aligned with respect to the longitudinally extending centerline 15 and the transversely extending centerline 17. In the specific embodiment of the invention shown in Figs. 1-4, the material-free zone 20 is substantially elliptical in shape and preferably has a length as measured along the longitudinally extending centerline 15 in the range of about 40 mm to about 160 mm and a width as measured along the transversely extending centerline 17 of about 10 mm to about 60 mm. The material-free zone 20 preferably extends over a surface area in the range of between 400 mm² and about 6000 mm².

As best seen in Fig. 4, the cover layer 12 includes a first region 22 located outside the area of the material-free zone 20 that is arranged in spaced relationship to the transfer layer 16 and the cover layer includes a second region 24 within the area defined by the material-free zone 20 that is arranged in surface to surface contact with the transfer layer 16. The surface to surface contact of the cover layer 12 with the transfer layer 16 essentially defines a gutter 29 in the body facing surface 11 of the napkin 10. The absorbent core 14 preferably has a thickness of between about 0.5 mm and about 20 mm. The depth of the gutter 29 is in the range of between about 0.5 mm and about 20 mm. The thickness and depth measurements set forth in this paragraph, and in this disclosure in general, may be determined by using a suitable thickness gauge such as the Mitutoyo Absolute Gauge or equivalent.

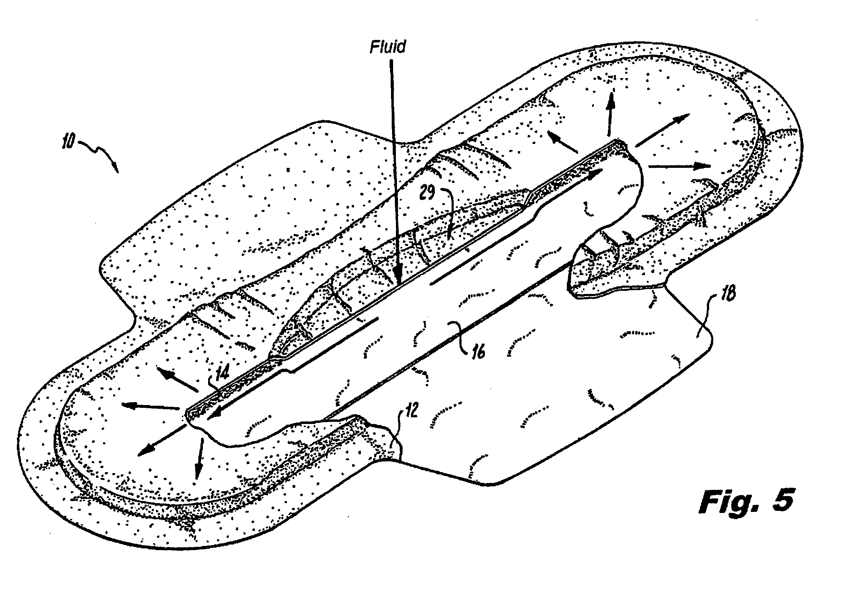

Reference is made to Fig. 5 which depicts the manner in which fluid is conveyed within the absorbent structure of a napkin 10 according to the present invention. As shown, the transfer layer 16 directly receives fluid from the cover layer 12 in the area of the material-free zone 20. The transfer layer 16 then wicks the fluid in the longitudinal and transverse directions of the napkin until the fluid can be conveyed upward and absorbed into the absorbent core 14.

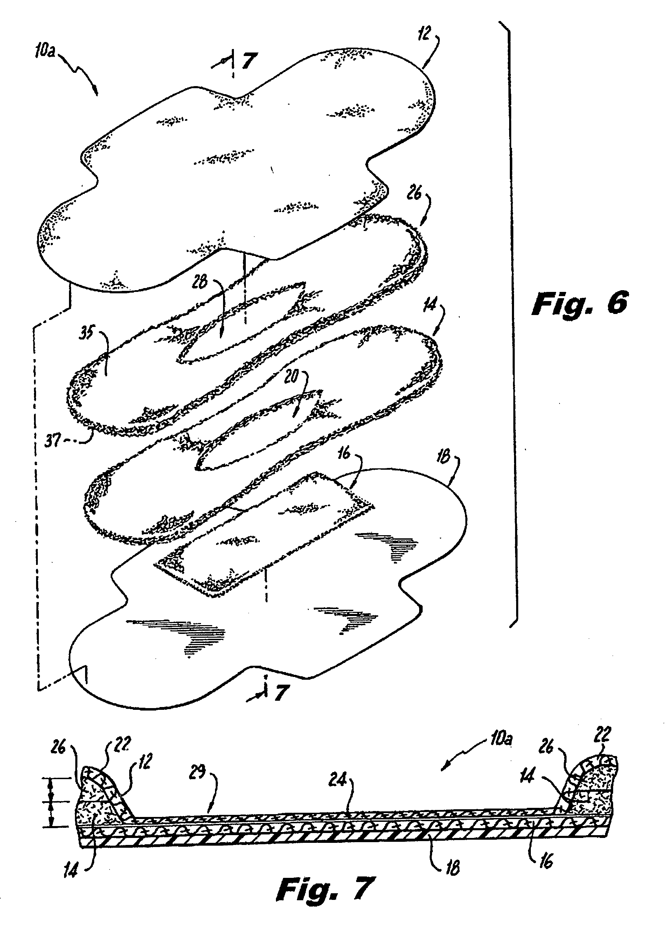

Reference is made to Fig. 6 which depicts an exploded view of a sanitary napkin 10a according to a second embodiment of the present invention. The sanitary napkin 10a is similar in structure to the sanitary napkin 10 described above but further includes a secondary absorbent core 26 arranged between the primary absorbent core 14 and the transfer layer 16. As shown, the secondary absorbent core 26 includes a material-free zone 28 that corresponds in size and shape to the material-free zone 20 of the primary absorbent core 14. The material-free zone 28 extends from an upper surface 35 of the secondary absorbent core 26 to a lower surface 37 of the secondary absorbent core 26.

Referring to Fig. 7, the cover layer 12 includes a first region 22 located outside the area of the material-free zones 20 and 28 that is arranged in spaced relationship to the transfer layer 16 and the cover layer includes a second region 24 within area of the material-free zones 20 and 28 that is arranged in surface to surface contact with the transfer layer 16. The surface to surface contact of the cover layer 12 with the transfer layer 16 essentially defines a gutter 29 in the body facing surface of the napkin 10. The primary absorbent core 14 and the secondary absorbent core 28 preferably each has a thickness of between about 0.5 mm and about 20 mm. The depth of each gutter 29 is in the range of between about 1.0 mm and about 40 mm.

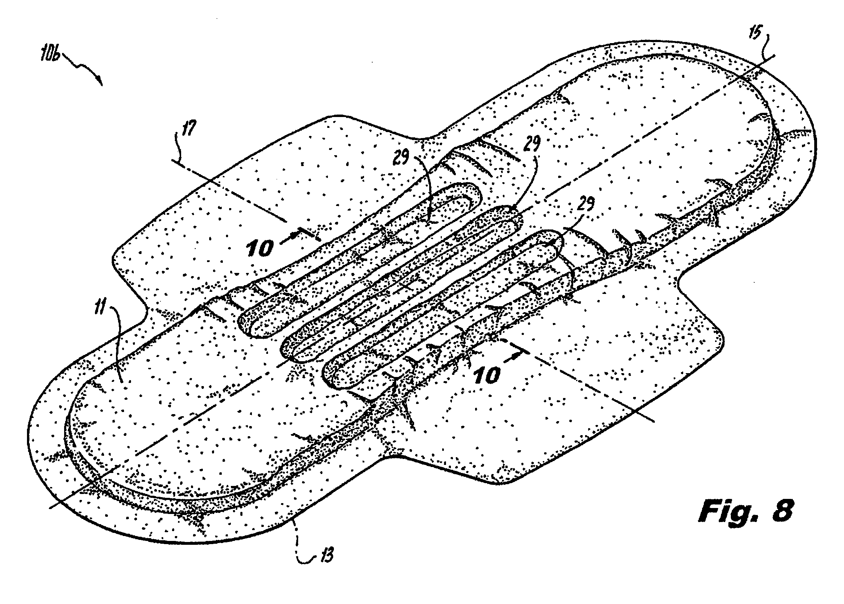

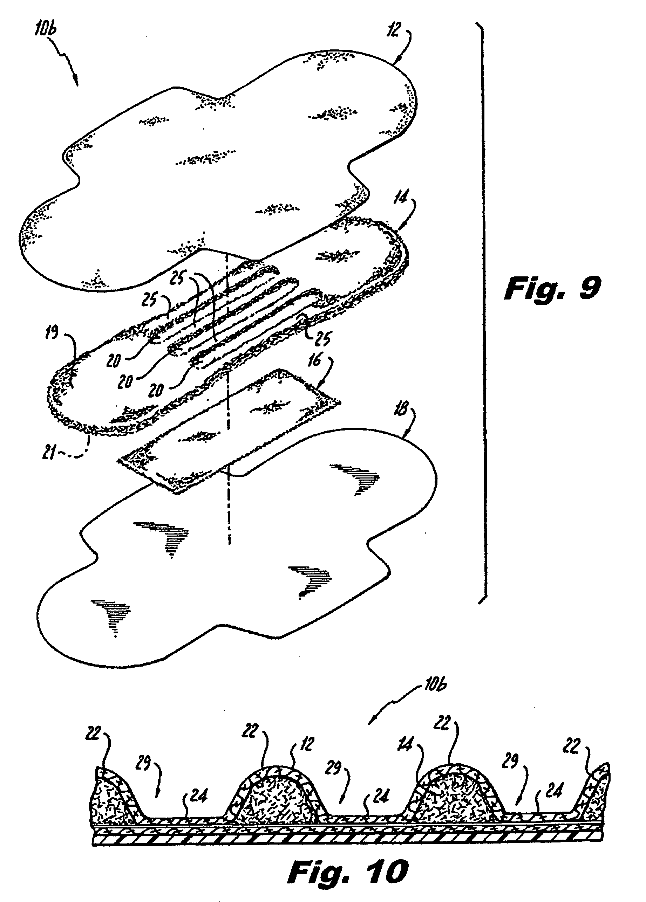

Reference is made to Figs. 8-10 which depict a sanitary napkin 10b according to a third embodiment of the present invention. As shown in Fig. 9, the sanitary napkin 10b includes a fluid permeable cover layer 12, an absorbent core 14, a transfer layer 16, and a fluid impermeable barrier layer 18. As shown in Fig. 9, the absorbent core 14 is arranged adjacent to the cover layer 12 and the transfer layer 16 is arranged between the absorbent core 14 and the barrier layer 18.

As best seen in the exploded view shown in Fig. 9, the absorbent core 14 includes a plurality of longitudinally extending material-free zones 20 that extend from an upper surface 19 of the absorbent core 14 to a lower surface 21 of the absorbent core 14. Each of the material-free zones 20 preferably has a width in the range of between 1 mm and about 10 mm and a length in the range of between about 50 mm and about 250 mm. Absorbent articles according to the third embodiment of the present invention preferably have between about 2 and about 7 longitudinally extending the material-free zones 20. Each of the material free zones 20 is spaced from an adjacent material-free zone 20 in the transverse direction by a distance from about 5 mm to about 30 mm. Each of the material-free zones 20 preferably extends over a surface area in the range of between about 50 mm² and about 4000 mm². In the particular embodiment of the invention shown in the Figs. 8-10 the material-free zones 20 are linear in shape, parallel to each other, and equally spaced.

The absorbent core 14 further includes a plurality of longitudinally extending beams 25, each of the beams 25 being arranged in spaced relationship to an adjacent beam 25 and each of the beams 25 being separated from an adjacent beam 25 by one of the material-free zones 20.

As best seen in Fig. 10, the cover layer 12 includes a plurality of first regions 22 that are arranged in spaced relationship to the transfer layer 16 and a plurality of second regions 24 that are arranged in surface to surface contact with the transfer layer 16. The surface to surface contact of the cover layer 12 with transfer layer 16 in the second regions 24 essentially define a plurality of longitudinally extending gutters 29 in the body facing surface 11 of the napkin 10 that are coextensive with the path of the material-free zones 20. The absorbent core 14 preferably has a thickness of between about 0.5 mm and about 20 mm. The depth of each gutter 29 is in the range of between about 0.5 mm and about 20 mm.

Although not shown in the Figures, the sanitary napkin 10b may be provided with a secondary absorbent core arranged between the primary core 14 and the transfer layer 16, the secondary absorbent core including a plurality of material-free zones that correspond in shape and size to the material-free zones of the primary core 14.

Although not shown in the Figures, the areas of the napkin in which the gutters 29 are located may be colored a different color than the remainder of the absorbent article. For example, the areas in which the gutters 29 are located may be colored blue while the remainder of the napkin is generally white. By coloring the gutters 29 a different color than the remainder of the napkin, the enhanced wicking characteristics provided by the gutters 29 are visually communicated to a potential user of the absorbent article. The color may be imparted to the napkin by providing a color (e.g., ink) to the cover layer 12 and/or the transfer layer 16 and/or the barrier layer 18.

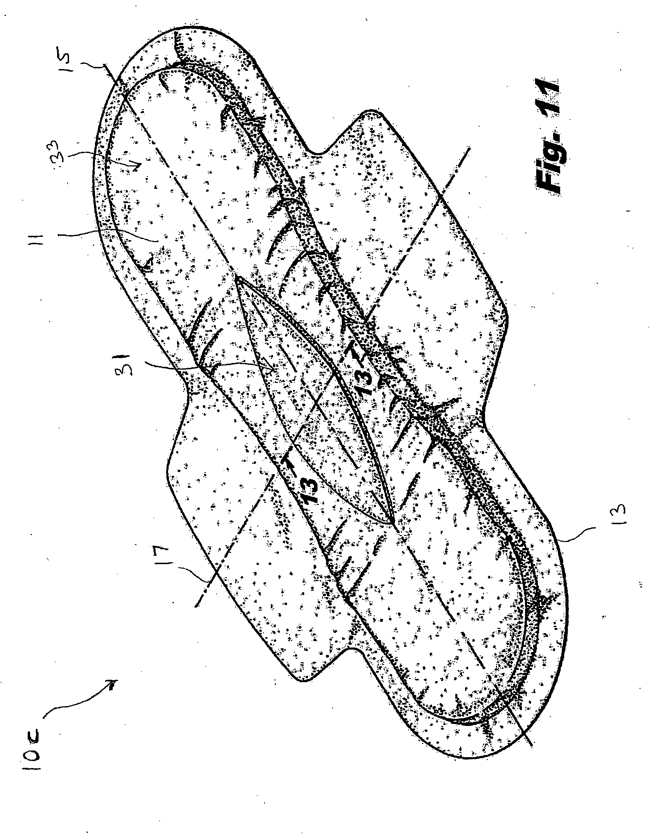

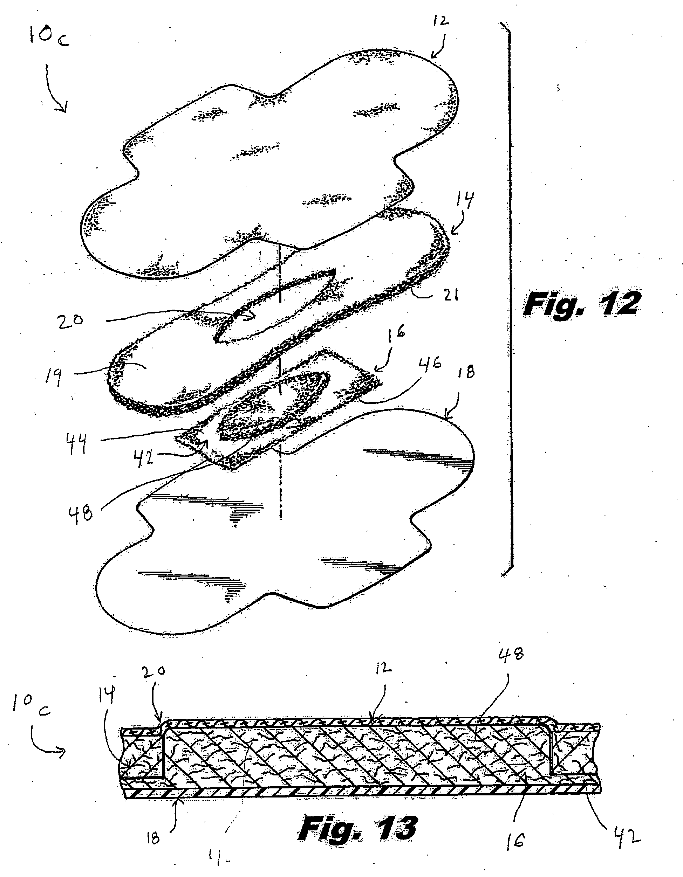

Reference is made to Figs. 11-13 which depicts a sanitary napkin 10c according to a fourth embodiment of the present invention. The sanitary napkin 10c is similar in structure to the sanitary napkin 10 described above. However in the embodiment of the invention shown in Figs. 11-13, the transfer layer 16 is provided with a planar portion 42 having an upper surface 44 and a lower surface 46 and a protrusion 48 that extends upwardly from the upper surface 44, as best seen in Fig. 12. In the specific embodiment of the invention shown in Figs. 11-13, the protrusion 48 is structured and arranged to align with the material free zone 20 of the absorbent core 14. More specifically, as seen in Fig. 13, the protrusion 48 is structured and arranged to be received within, and extend upwardly into, the material free zone 20 of the absorbent core 14.

In the specific embodiment of the invention shown in Figs. 11-13 the protrusion 48 is generally elliptical in shape and preferably has a length as measured along the longitudinally extending centerline in the range of about 40 mm to about 160 mm and a width as measured along the transversely extending centerline of about 10 mm to about 60 mm. The protrusion 48 preferably extends over a surface area in the range of between about 400 mm² to about 6000 mm².

The protrusion 48 preferably has a height that is greater than the distance between the upper surface 19 of the absorbent core to the lower surface 21 of the absorbent core. Preferably the height of the protrusion 48 is in the range of between about 0.5 mm to about 50 mm, more preferably in the range of between about 2.0 mm and 35 mm and most preferably in the range of between about 2.5 mm and 30 mm. The term "height of the protrusion" as used herein means the distance the protrusion 48 extends above the upper surface 44 of the planar portion 42. Since the protrusion 48 has a height that is greater than the distance between the upper surface 19 of the absorbent core to the lower surface 21 of the absorbent core, the protrusion 48 functions to define a raised area 31 that extends upwardly from the planar portion 33 of the napkin 10c, as best seen in Fig. 11. As shown in Fig. 11 the raised area 31 has a geometry that corresponds in shape to the shape of the protrusion 48.

The planar portion 42 and the protrusion 48 of the transfer layer 16 may be formed by any conventional method known to those of skill in the art. For example, the transfer layer 16 may be compressed in area defining the planar portion 42 and non-compressed in the area defining the protrusion 48.

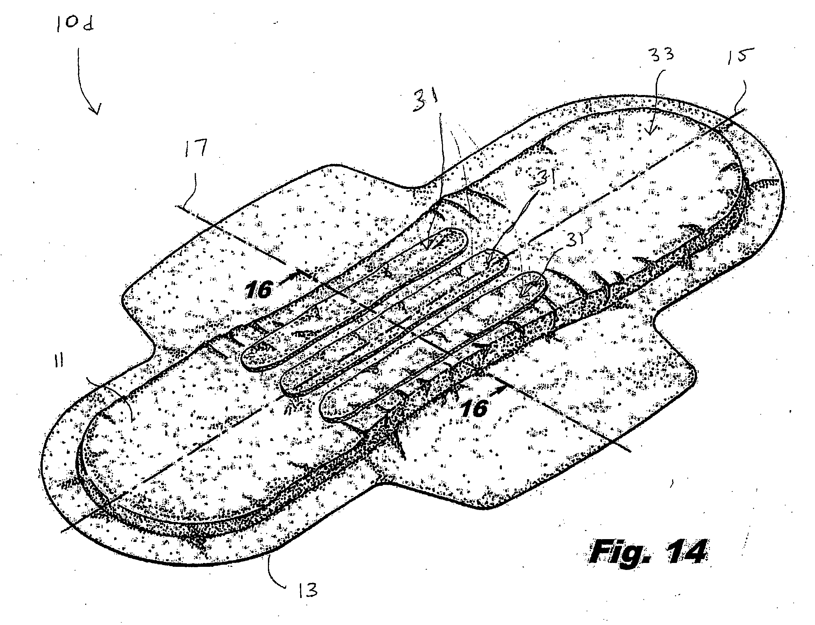

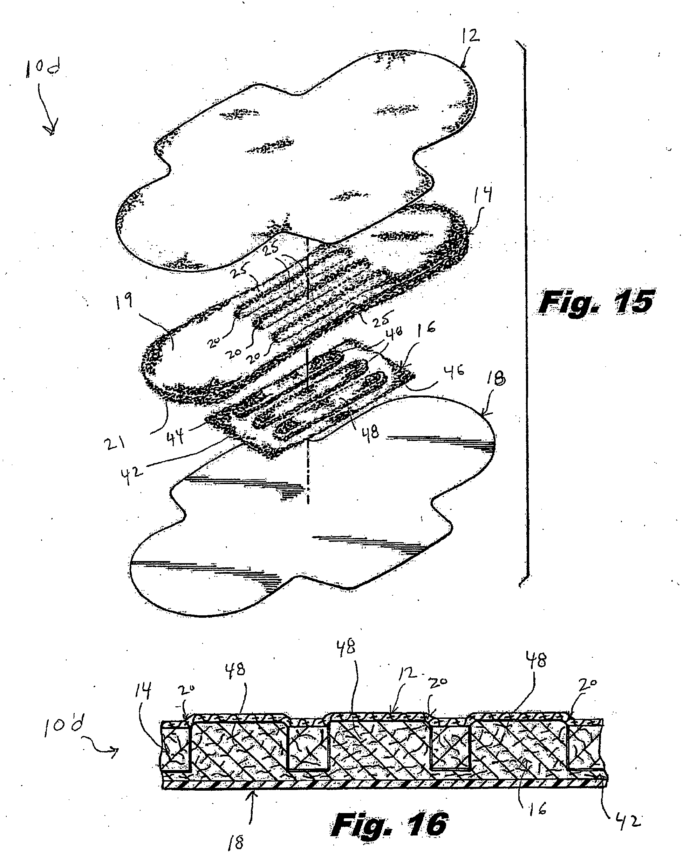

Reference is made to Figs. 14-16 which depicts a sanitary napkin 10d according to a fifth embodiment of the present invention. The sanitary napkin 10d is similar in structure to the sanitary napkin 10b described above. However in the embodiment of the invention shown in Figs. 14-16, the transfer layer 16 is provided with a planar portion 42 having an upper surface 44 and a lower surface 46 and a plurality of protrusions 48 that extend upwardly from the upper surface 44, as best seen in Fig. 15. In the specific embodiment of the invention shown in Figs. 14-16, each of the protrusions 48 is structured and arranged to align with one of the plurality of material free zones 20 in the absorbent core 14. More specifically, as seen in Fig. 16, each protrusion 48 is structured and arranged to be received within, and extend upwardly into, one of the material free zones 20 of the absorbent core 14.

In the specific embodiment of the invention shown in Figs. 14-16 each protrusion 48 has a width in the range of between about 1 mm and about 10 mm and a length in the range of between about 50 mm and 250 mm. Absorbent articles according to this embodiment of the invention preferably have between about 2 and about 7 longitudinally extending protrusions 48. Each protrusion 48 is spaced from an adjacent protrusion by a distance from about 5 mm to about 30 mm. Each protrusion preferably extends over a surface area in the range of between about 50 mm² to about 4000 mm². In the particular embodiment of the invention shown in Figs. 14-16 the protrusions 48 are linear in shape, parallel to each other, and equally spaced.

Preferably each protrusion 48 has a height that is greater than the distance between the upper surface 19 of the absorbent core to the lower surface 21 of the absorbent core. Preferably the height of each protrusion 48 is in the range of between about 0.5 mm to about 50 mm, more preferably in the range of between about 2.0 mm and 35 mm, and most preferably in the range of between about 2.5 mm and 30 mm. The term "height of the protrusion" as used herein means the distance the protrusion extends above the upper surface 44 of the planar portion 42. Since each of the protrusions 48 has a height that is greater than the distance between the upper surface 19 of the absorbent core to the lower surface 21 of the absorbent core, the protrusions 48 function to define a plurality of raised areas 31 that extend upwardly from the planar portion 33 of the napkin 10d, as best seen in Fig. 14. As shown in Fig. 14 each of the raised areas 31 has a geometry that corresponds in shape to the shape of a corresponding protrusion 48.

The planar portion 42 and each of the protrusions 48 may be formed by any conventional method known to those of skill in the art. For example, the transfer layer 16 may be compressed in the area defining the planar portion 42 and non-compressed in the areas defining the protrusions 48.

### Cover Layer

The cover layer 12 may be a relatively low density, bulky, high-loft non-woven web material. The cover layer 12 may be composed of only one type of fiber, such as polyester or polypropylene or it may include a mixture of more than one fiber. The cover may be composed of bi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Preferably, the cover layer 12 has a basis weight in the range of about 10 gsm to about 75 gsm.

Bi-component fibers may be made up of a polyester layer and a polyethylene sheath. The use of appropriate bi-component materials results in a fusible non-woven fabric. Examples of such fusible fabrics are described in U.S. Pat. No. 4,555,430 issued Nov. 26, 1985 to Chicopee. Using a fusible fabric increases the ease with which the cover layer may be mounted to the absorbent layers of the article and/or to the barrier layer.

The cover layer 12 preferably has a relatively high degree of wettability, although the individual fibers comprising the cover may not be particularly hydrophilic. The cover material should also contain a great number of relatively large pores. This is because the cover layer 12 is intended to take-up body fluid rapidly and transport it away from the body and the point of deposition. Therefore, the cover layer contributes little to the time taken for the napkin to absorb a given quantity of liquid (penetration time).

Advantageously, the fibers which make up the cover layer 12 should not lose their physical properties when they are wetted, in other words they should not collapse or lose their resiliency when subjected to water or body fluid. The cover layer 12 may be treated to allow fluid to pass through it readily. The cover layer 12 also functions to transfer the fluid quickly to the underlying layers of the napkin. Thus, the cover layer 12 is advantageously wettable, hydrophilic and porous. When composed of synthetic hydrophobic fibers such as polyester or bi-component fibers, the cover layer 12 may be treated with a surfactant to impart the desired degree of wettability.

Alternatively, the cover layer 12 can be made of a polymer film having large pores. Because of such high porosity, the film accomplishes the function of quickly transferring body fluid to the underlying absorbent layers.

The cover layer 12 may be attached to the underlying absorbent core 14, transfer layer 16, and/or the barrier layer 18, by adhesion and/or other suitable means know to those of skill in the art.

### Absorbent Core

In one embodiment, the absorbent core 14 is a blend or mixture of cellulosic fibers and superabsorbent disposed therein. Cellulosic fibers that can be used in the absorbent core 14 are well known in the art and include wood pulp, cotton, flax and peat moss. Wood pulp is preferred. Pulps can be obtained from mechanical or chemi-mechanical, sulfite, kraft, pulping reject materials, organic solvent pulps, etc. Both softwood and hardwood species are useful. Softwood pulps are preferred. It is not necessary to treat cellulosic fibers with chemical debonding agents, cross-linking agents and the like for use in the present material. Some portion of the pulp may be chemically treated as discussed in U.S. Pat. No. 5,916,670 to improved flexibility of the product. Flexibility of the material may also be improved by mechanically working the material or tenderizing the material.

The absorbent core 14 can contain any superabsorbent polymer (SAP) which are well known in the art. For the purposes of the present invention, the term "superabsorbent polymer" (or "SAP") refers to materials which are capable of absorbing and retaining at least about 10 times their weight in body fluids under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. of Osaka, Japan, under the designation of SA70N and products offered by Stockhausen Inc.. In a specific example, the absorbent core is a material containing from 95% to about 40% percent cellulosic fiber by weight, and about 5% to about 60% SAP by weight.

In one specific embodiment of the invention, the absorbent core 14 is constructed from a mixture of fluff pulp, commercially available as RAYFLOC J-LD-E from Rayonier Products, Jessup, Georgia, and superabsorbent polymer commercially available under the designation SA70N from Sumitomo Seika Chemicals Co., Ltd. Of Osaka, Japan, the mixture including 94 % fluff pulp by weight and 6% superabsorbent polymer by weight.

Materials particularly suitable for use as the absorbent core preferably have a basis weight in the range from about 300 gsm (g/m²) to 1000 gsm, a thickness in the range of about 0.5 mm to 20 mm, and a density in the range of about 0.015 g/cc to 2 g/cc.

### Transfer Layer

Adjacent to the barrier layer 18 layer on its inner side is the transfer layer 16. The transfer provides the means of receiving body fluid from the cover layer 12 and holding it until the absorbent core 14 has an opportunity to absorb the fluid, and therefore serves as a fluid transfer or acquisition layer. In addition the transfer layer 16 functions to wick the fluid in the longitudinal and transverse directions of the napkin so that the full absorbent capacity of the napkin is utilized.

The transfer layer 16 is, preferably, has a larger proportion of smaller pores than the cover layer 12. These attributes allow the transfer layer 16 to contain body fluid and hold it away from the outer side of the cover layer 12, thereby preventing the fluid from re-wetting the cover layer 12 and its surface.

The transfer layer 16 may be composed of fibrous materials, such as wood pulp, polyester, rayon, flexible foam, or the like, or combinations thereof. Preferably, the transfer layer 16 is free of any superabsorbent polymer (SAP). The transfer layer 16 may also comprise thermoplastic fibers for the purpose of stabilizing the layer and maintaining its structural integrity. The transfer layer 16 may be treated with surfactant on one or both sides in order to increase its wettability, although generally the transfer layer 16 is relatively hydrophilic and may not require treatment. The transfer layer 16 is preferably bonded on both sides to the adjacent layers, i.e. the absorbent core 14 and the barrier layer 18.

### Barrier Layer

Underlying the transfer layer 16 is a barrier layer 18 comprising liquid-impervious film material so as to prevent liquid from egressing the sanitary napkin and staining the wearer's undergarment. The barrier layer 18 is preferably made of polymeric film, although it may be made of liquid impervious, air-permeable material such as repellent-treated non-woven or micropore films or foams.

The barrier layer 18 may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet. The cover layer 12 and the barrier layer 18 are preferably joined along their marginal portions so as to form an enclosure or flange seal that maintains the transfer layer 16 and absorbent core 14 captive. The joint may be made by means of adhesives, heat-bonding, ultrasonic bonding, radio frequency sealing, mechanical crimping, and the like and combinations thereof.

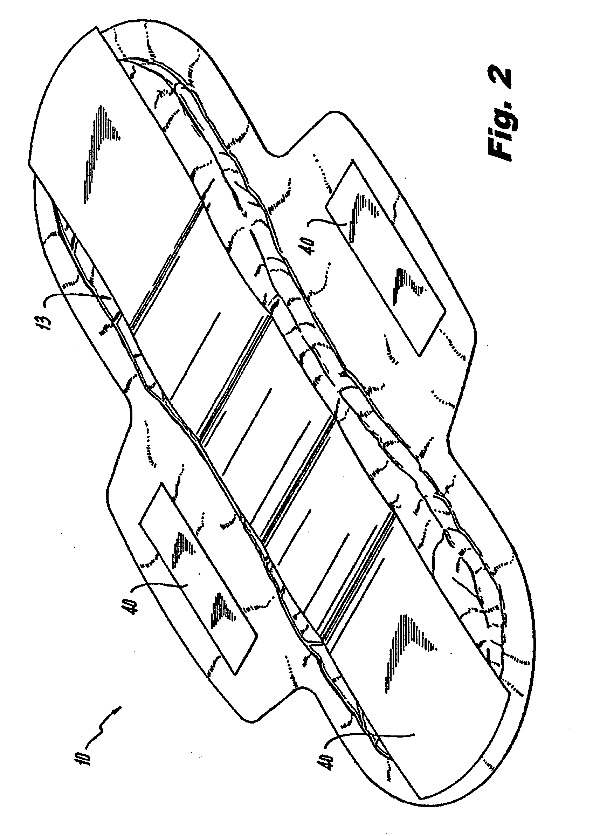

Positioning adhesive may be applied to a garment facing surface 13 of the barrier layer 18 for securing the napkin 10 to a garment during use. As seen in Fig. 2, the positioning adhesive may be covered with removable release paper 40 so that the positioning adhesive is covered by the removable release paper 40 prior to use.

Absorbent articles of this invention may or may not include wings, flaps or tabs for securing the absorbent article to an undergarment. Wings, also called, among other things, flaps or tabs, and their use in sanitary protection articles is described in U.S. Patent. No. 4,687,478 to Van Tilburg; U.S. Patent No. 4,589,876 also to Van Tilburg, U.S. Patent No. 4,900,320 to McCoy, and U.S. Patent No. 4,608,047 to Mattingly. As disclosed in the above documents, wings are generally speaking flexible and configured to be folded over the edges of the underwear so that the wings are disposed between the edges of the underwear.

## Claims

1. An absorbent article comprising:
a longitudinally extending centerline;
a transversely extending centerline;
a liquid permeable cover layer having a body facing surface;
a liquid impermeable barrier layer;
an absorbent core arranged adjacent to the cover layer, the absorbent core including an upper surface and a lower surface and at least one material-free zone extending from the upper surface to the lower surface;
a transfer layer arranged between the core and the barrier layer, the transfer layer including a planar portion having an upper surface and a lower surface and at least one protrusion extending upwardly from the upper surface;
wherein the cover layer includes at least one first region arranged in spaced relationship to the transfer layer and at least one second region arranged in surface to surface contact with the transfer layer;
wherein the at least one protrusion is structured and arranged to be received within, and extend upwardly into the at least one material free zone; and
wherein the at least one protrusion has a height that is greater than a distance between the upper surface of the absorbent core and the lower surface of the absorbent core.

2. An absorbent article according to claim 1 comprising:
a longitudinally extending centerline;
a transversely extending centerline;
a liquid permeable cover layer having a body facing surface;
a liquid impermeable barrier layer;
an absorbent core arranged adjacent to the cover layer, the absorbent core including an upper surface and a lower surface and a material-free zone extending from the upper surface to the lower surface;
a transfer layer arranged between the core and the barrier layer, the transfer layer including a planar portion having an upper surface and a lower surface and a protrusion extending upwardly from the upper surface;
wherein the cover layer includes a first region arranged in spaced relationship to the transfer layer and a second region arranged in surface to surface contact with the transfer layer;
wherein the protrusion is structured and arranged to be received within, and extend upwardly into the material free zone; and
wherein the protrusion has a height that is greater than a distance between the upper surface of the absorbent core and the lower surface of the absorbent core.

3. The absorbent article according to claim 2, wherein the material-free zone is centrally aligned with respect to the longitudinally extending centerline and the transversely extending centerline.

4. The absorbent article according to claim 2 or claim 3, wherein the material-free zone extends over an area between about 400 mm² and about 6000 mm².

5. The absorbent article according to any one of claims 2 to 4, wherein the material-free zone is substantially elliptical in shape and has a length as measured along the longitudinally extending centerline in the range of about 40 mm to about 250 mm and a width as measured along the transversely extending centerline of about 10 mm to about 60 mm.

6. The absorbent article according to any one of claims 2 to 5, wherein the protrusion extends over an area between about 400 mm² and about 6000 mm².

7. An absorbent article according to claim 1 comprising:
a longitudinally extending centerline;
a transversely extending centerline;
a liquid permeable cover layer having a body facing surface;
a liquid impermeable barrier layer;
an absorbent core arranged adjacent to the cover layer;
a transfer layer arranged between the core and the barrier layer, the transfer layer including a planar portion having an upper surface and a lower surface and a plurality of protrusions extending upwardly from the upper surface;
wherein the absorbent core includes an upper surface and a lower surface, the absorbent core comprising a plurality of beams and a plurality of material-free zones, each of the beams arranged in a spaced relationship to an adjacent beam and each of the beams being separated from an adjacent beam by a material-free zone, each of the material-free zones extending from the upper surface to the lower surface;
wherein the cover layer includes a plurality of first regions arranged in spaced relationship to the transfer layer and a plurality of second regions, each of the second regions located between two adjacent beams and arranged in surface to surface contact with the transfer layer;
wherein each one of the plurality of protrusions is structured and arranged to be received within, and extend upwardly into one of the plurality of material free zones; and
wherein each protrusion has a height that is greater than a distance between the upper surface of the absorbent core and the lower surface of the absorbent core.

8. The absorbent article according to claim 7, wherein each of the material-free zones has a width in the range of between about 1 mm and about 10 mm and a length in the range of between about 40 mm and about 250 mm.

9. The absorbent article according to claim 7 or claim 8, wherein the absorbent article includes between 2 and about 7 longitudinally extending material-free zones and wherein each of the material free zones is spaced from an adjacent material-free zone in the transverse direction by a distance of from about 5 mm to about 30 mm.

10. The absorbent article according to any one of claims 7 to 9, wherein each of the material-free zones extend over a surface area in the range of between 50 mm² and about 4000 mm².

11. The absorbent article according to any one of claims 7 to 10, wherein each protrusion has a width in the range of between about 1 mm and about 10 mm and a length in the range of between about 40 mm and about 250 mm.

12. The absorbent article according to any one of claims 7 to 11, wherein the absorbent article includes between 2 and about 7 protrusions and each protrusion is spaced from an adjacent protrusion in the transverse direction by a distance of from about 5 mm to about 30 mm.

13. The absorbent article according to any one of claims 7 to 12, wherein each of the protrusions extends over a surface area in the range of between 50 mm² and about 4000 mm².

14. The absorbent article according to any preceding claim, wherein each protrusion has a height in the range of between about 0.5 mm and about 50 mm.

15. The absorbent article according to any preceding claim, wherein each protrusion has a height in the range of between about 1.0 mm and about 35 mm, or
wherein each protrusion has a height in the range of between about 1.5 mm and about 30 mm.

## Patentansprüche

1. Saugfähiger Gegenstand, umfassend:
eine längsverlaufende Mittellinie;
eine querverlaufende Mittellinie;
eine flüssigkeitsdurchlässige Deckschicht, welche eine dem Körper zugewandte Fläche aufweist;
eine flüssigkeitsundurchlässige Barriereschicht;
einen saugfähigen Kern, der angrenzend zu der Deckschicht angeordnet ist, wobei der saugfähige Kern eine obere Fläche und eine untere Fläche und mindestens eine materialfreie Zone umfasst, die von der oberen Fläche zu der unteren Fläche verläuft;
eine Transferschicht, die zwischen dem Kern und der Barriereschicht angeordnet ist, wobei die Transferschicht einen planaren Teil aufweist, der eine obere Fläche und eine untere Fläche und mindestens einen Vorsprung aufweist, der von der oberen Fläche aus nach oben verläuft;
wobei die Deckschicht mindestens eine erste Region, die in beabstandeter Beziehung zu der Transferschicht angeordnet ist, und mindestens eine zweite Region, die von Fläche zu Fläche in Kontakt mit der Transferschicht angeordnet ist, umfasst;
wobei der mindestens eine Vorsprung strukturiert und angeordnet ist, um in der mindestens einen materialfreien Zone aufgenommen zu werden und nach oben in die mindestens eine materialfreie Zone zu verlaufen; und
wobei der mindestens eine Vorsprung eine Höhe aufweist, die größer ist als ein Abstand zwischen der oberen Fläche des saugfähigen Kerns und der unteren Fläche des saugfähigen Kerns.

2. Saugfähiger Gegenstand nach Anspruch 1, umfassend:
eine längsverlaufende Mittellinie;
eine querverlaufende Mittellinie;
eine flüssigkeitsdurchlässige Deckschicht, welche eine dem Körper zugewandte Fläche aufweist;
eine flüssigkeitsundurchlässige Barriereschicht;
einen saugfähigen Kern, der angrenzend zu der Deckschicht angeordnet ist, wobei der saugfähige Kern eine obere Fläche und eine untere Fläche und eine materialfreie Zone umfasst, die von der oberen Fläche zu der unteren Fläche verläuft;
eine Transferschicht, die zwischen dem Kern und der Barriereschicht angeordnet ist, wobei die Transferschicht einen planaren Teil aufweist, der eine obere Fläche und eine untere Fläche und einen Vorsprung aufweist, der von der oberen Fläche aus nach oben verläuft;
wobei die Deckschicht eine erste Region, die in beabstandeter Beziehung zu der Transferschicht angeordnet ist, und eine zweite Region, die von Fläche zu Fläche in Kontakt mit der Transferschicht angeordnet ist, umfasst;
wobei der Vorsprung strukturiert und angeordnet ist, um in der materialfreien Zone aufgenommen zu werden und nach oben in die materialfreie Zone zu verlaufen; und
wobei der Vorsprung eine Höhe aufweist, die größer ist als ein Abstand zwischen der oberen Fläche des saugfähigen Kerns und der unteren Fläche des saugfähigen Kerns.

3. Saugfähiger Gegenstand nach Anspruch 2, wobei die materialfreie Zone mittig in Bezug zu der längsverlaufenden Mittellinie und der querverlaufenden Mittellinie ausgerichtet ist.

4. Saugfähiger Gegenstand nach Anspruch 2 oder Anspruch 3, wobei die materialfreie Zone über eine Fläche zwischen etwa 400 mm² und etwa 6000 mm² verläuft.

5. Saugfähiger Gegenstand nach einem der Ansprüche 2 bis 4, wobei die materialfreie Zone eine im Wesentlichen elliptische Form aufweist und eine Länge, wie entlang der längsverlaufenden Mittellinie gemessen, im Bereich von etwa 40 mm bis etwa 250 mm und eine Breite, wie entlang der querverlaufenden Mittellinie gemessen, von etwa 10 mm bis etwa 60 mm hat.

6. Saugfähiger Gegenstand nach einem der Ansprüche 2 bis 5, wobei der Vorsprung über eine Fläche zwischen etwa 400 mm² und etwa 6000 mm² verläuft.

7. Saugfähiger Gegenstand nach Anspruch 1, umfassend:
eine längsverlaufende Mittellinie;
eine querverlaufende Mittellinie;
eine flüssigkeitsdurchlässige Deckschicht, welche eine dem Körper zugewandte Fläche aufweist;
eine flüssigkeitsundurchlässige Barriereschicht;
einen saugfähigen Kern, der angrenzend zu der Deckschicht angeordnet ist;
eine Transferschicht, die zwischen dem Kern und der Barriereschicht angeordnet ist, wobei die Transferschicht einen planaren Teil aufweist, der eine obere Fläche und eine untere Fläche und mehrere Vorsprünge aufweist, die von der oberen Fläche aus nach oben verlaufen;
wobei der saugfähige Kern eine obere Fläche und eine untere Fläche aufweist, wobei der saugfähige Kern mehrere Holmen und mehrere materialfreie Zonen aufweist, wobei jeder der Holmen in beabstandeter Beziehung zu einem angrenzenden Holm angeordnet ist und jeder der Holmen von einem angrenzenden Holm durch eine materialfreie Zone getrennt ist, wobei jede der materialfreien Zonen von der oberen Fläche zu der unteren Fläche verläuft;
wobei die Deckschicht mehrere erste Regionen, die in beabstandeter Beziehung zu der Transferschicht angeordnet sind, und mehrere zweite Regionen umfasst, wobei sich jede der zweiten Regionen zwischen zwei angrenzenden Holmen befindet und von Fläche zu Fläche in Kontakt mit der Transferschicht angeordnet ist;
wobei jeder der mehreren Vorsprünge strukturiert und angeordnet ist, um in einer der mehreren materialfreien Zonen aufgenommen zu werden und nach oben in eine der mehreren materialfreien Zonen zu verlaufen; und
wobei jeder Vorsprung eine Höhe aufweist, die größer ist als ein Abstand zwischen der oberen Fläche des saugfähigen Kerns und der unteren Fläche des saugfähigen Kerns.

8. Saugfähiger Gegenstand nach Anspruch 7, wobei jede der materialfreien Zonen eine Breite im Bereich zwischen etwa 1 mm und etwa 10 mm und eine Länge im Bereich zwischen etwa 40 mm und etwa 250 mm aufweist.

9. Saugfähiger Gegenstand nach Anspruch 7 oder Anspruch 8, wobei der saugfähige Gegenstand zwischen 2 und etwa 7 längsverlaufende materialfreie Zonen umfasst und wobei jede der materialfreien Zonen von einer angrenzenden materialfreien Zone in der Querrichtung mit einem Abstand von etwa 5 mm bis etwa 30 mm beabstandet ist.

10. Saugfähiger Gegenstand nach einem der Ansprüche 7 bis 9, wobei jede der materialfreien Zonen über eine Fläche im Bereich zwischen 50 mm² und etwa 4000 mm² verläuft.

11. Saugfähiger Gegenstand nach einem der Ansprüche 7 bis 10, wobei jeder Vorsprung eine Breite im Bereich zwischen etwa 1 mm und etwa 10 mm und eine Länge im Bereich zwischen etwa 40 mm und etwa 250 mm aufweist.

12. Saugfähiger Gegenstand nach einem der Ansprüche 7 bis 11, wobei der saugfähige Gegenstand zwischen 2 und etwa 7 Vorsprünge aufweist und jeder Vorsprung von einem angrenzenden Vorsprung in der Querrichtung mit einem Abstand von etwa 5 mm bis etwa 30 mm beabstandet ist.

13. Saugfähiger Gegenstand nach einem der Ansprüche 7 bis 12, wobei jeder der Vorsprünge über eine Fläche im Bereich zwischen 50 mm² und etwa 4000 mm² verläuft.

14. Saugfähiger Gegenstand nach einem der vorhergehenden Ansprüche, wobei jeder Vorsprung eine Höhe im Bereich zwischen etwa 0,5 mm und etwa 50 mm aufweist.

15. Saugfähiger Gegenstand nach einem der vorhergehenden Ansprüche, wobei jeder Vorsprung eine Höhe im Bereich zwischen etwa 1,0 mm und etwa 35 mm aufweist oder wobei jeder Vorsprung eine Höhe im Bereich zwischen etwa 1,5 mm und etwa 30 mm aufweist.

## Revendications

1. Article absorbant comprenant :
une ligne médiane s'étendant longitudinalement ;
une ligne médiane s'étendant transversalement ;
une couche de recouvrement perméable aux liquides comportant une surface faisant face au corps ;
une couche barrière imperméable aux liquides ;
une partie centrale absorbante disposée de façon adjacente à la couche de recouvrement, la partie centrale absorbante comprenant une surface supérieure et une surface inférieure et au moins une zone dépourvue de matériau s'étendant de la surface supérieure à la surface inférieure ;
une couche de transfert disposée entre la partie centrale et la couche barrière, la couche de transfert comprenant une partie plane comportant une surface supérieure et une surface inférieure et au moins une partie saillante s'étendant vers le haut à partir de la surface supérieure ;
dans lequel la couche de recouvrement comprend au moins une première région disposée de façon à être espacée de la couche de transfert et au moins une deuxième région disposée en contact surface contre surface avec la couche de transfert ;
dans lequel ladite au moins une partie saillante est structurée et disposée pour se mettre en place à l'intérieur de, et s'étendre vers le haut dans, ladite au moins une zone dépourvue de matériau ; et
dans lequel ladite au moins une partie saillante a une hauteur qui est supérieure à une distance entre la surface supérieure de la partie centrale absorbante et la surface inférieure de la partie centrale absorbante.

2. Article absorbant selon la revendication 1, comprenant :
une ligne médiane s'étendant longitudinalement ;
une ligne médiane s'étendant transversalement ;
une couche de recouvrement perméable aux liquides comportant une surface faisant face au corps ;
une couche barrière imperméable aux liquides ;
une partie centrale absorbante disposée de façon adjacente à la couche de recouvrement, la partie centrale absorbante comprenant une surface supérieure et une surface inférieure et une zone dépourvue de matériau s'étendant de la surface supérieure à la surface inférieure ;
une couche de transfert disposée entre la partie centrale et la couche barrière, la couche de transfert comprenant une partie plane comportant une surface supérieure et une surface inférieure et une partie saillante s'étendant vers le haut à partir de la surface supérieure ;
dans lequel la couche de recouvrement comprend une première région disposée de façon à être espacée de la couche de transfert et une deuxième région disposée en contact surface contre surface avec la couche de transfert ;
dans lequel la partie saillante est structurée et disposée pour se mettre en place à l'intérieur de, et s'étendre vers le haut dans, la zone dépourvue de matériau ; et
dans lequel la partie saillante a une hauteur qui est supérieure à une distance entre la surface supérieure de la partie centrale absorbante et la surface inférieure de la partie centrale absorbante.

3. Article absorbant selon la revendication 2, dans lequel la zone dépourvue de matériau est alignée centralement par rapport à la ligne médiane s'étendant longitudinalement et à la ligne médiane s'étendant transversalement.

4. Article absorbant selon la revendication 2 ou la revendication 3, dans lequel la zone dépourvue de matériau s'étend sur une surface d'environ 400 mm² à environ 6 000 mm².

5. Article absorbant selon l'une quelconque des revendications 2 à 4, dans lequel la zone dépourvue de matériau est de forme sensiblement elliptique et a une longueur, mesurée le long de la ligne médiane s'étendant longitudinalement, d'environ 40 mm à environ 250 mm et une largeur, mesurée le long de la ligne médiane s'étendant transversalement, d'environ 10 mm à environ 60 mm.

6. Article absorbant selon l'une quelconque des revendications 2 à 5, dans lequel la partie saillante s'étend sur une surface d'environ 400 mm² à environ 6 000 mm².

7. Article absorbant selon la revendication 1, comprenant :
une ligne médiane s'étendant longitudinalement ;
une ligne médiane s'étendant transversalement ;
une couche de recouvrement perméable aux liquides comportant une surface faisant face au corps ;
une couche barrière imperméable aux liquides ;
une partie centrale absorbante disposée de façon adjacente à la couche de recouvrement ;
une couche de transfert disposée entre la partie centrale et la couche barrière, la couche de transfert comprenant une partie plane comportant une surface supérieure et une surface inférieure et une pluralité de parties saillantes s'étendant vers le haut à partir de la surface supérieure ;
dans lequel la partie centrale absorbante comprend une surface supérieure et une surface inférieure, la partie centrale absorbante comprenant une pluralité d'éléments en forme de barres et une pluralité de zones dépourvues de matériau, chacun des éléments en forme de barres étant disposé de façon à être espacé d'un élément en forme de barre adjacent et chacun des éléments en forme de barres étant séparé d'un élément en forme de barre adjacent par une zone dépourvue de matériau, chacune des zones dépourvues de matériau s'étendant de la surface supérieure à la surface inférieure ;
dans lequel la couche de recouvrement comprend une pluralité de premières régions disposées de façon à être espacées de la couche de transfert et une pluralité de deuxièmes régions, chacune des deuxièmes régions étant située entre deux éléments en forme de barres adjacents et disposée en contact surface contre surface avec la couche de transfert ;
dans lequel chacune de la pluralité de parties saillantes est structurée et disposée pour se mettre en place à l'intérieur de, et s'étendre vers le haut dans, une de la pluralité de zones dépourvues de matériau ; et
dans lequel chaque partie saillante a une hauteur qui est supérieure à une distance entre la surface supérieure de la partie centrale absorbante et la surface inférieure de la partie centrale absorbante.

8. Article absorbant selon la revendication 7, dans lequel chacune des zones dépourvues de matériau a une largeur d'environ 1 mm à environ 10 mm et une longueur d'environ 40 mm à environ 250 mm.

9. Article absorbant selon la revendication 7 ou la revendication 8, l'article absorbant comprenant 2 à environ 7 zones dépourvues de matériau s'étendant longitudinalement et chacune des zones dépourvues de matériau étant espacée d'une zone dépourvue de matériau adjacente dans la direction transversale d'une distance d'environ 5 mm à environ 30 mm.

10. Article absorbant selon l'une quelconque des revendications 7 à 9, dans lequel chacune des zones dépourvues de matériau s'étend sur une aire de surface de 50 mm² à environ 4 000 mm².

11. Article absorbant selon l'une quelconque des revendications 7 à 10, dans lequel chaque partie saillante a une largeur d'environ 1 mm à environ 10 mm et une longueur d'environ 40 mm è environ 250 mm.

12. Article absorbant selon l'une quelconque des revendications 7 à 11, l'article absorbant comprenant 2 à environ 7 parties saillantes et chaque partie saillante étant espacée d'une partie saillante adjacente dans la direction transversale d'une distance d'environ 5 mm à environ 30 mm.

13. Article absorbant selon l'une quelconque des revendications 7 à 12, dans lequel chacune des parties saillantes s'étend sur une aire de surface de 50 mm² à environ 4 000 mm².

14. Article absorbant selon l'une quelconque des revendications précédentes, dans lequel chaque partie saillante a une hauteur d'environ 0,5 mm à environ 50 mm.

15. Article absorbant selon l'une quelconque des revendications précédentes, dans lequel chaque partie saillante a une hauteur d'environ 1,0 mm à environ 35 mm, ou dans lequel chaque partie saillante a une hauteur d'environ 1,5 mm à environ 30 mm.
